# EUROPEAN PATENT SPECIFICATION

(11) **EP 1 962 723 B1**
(45) Date of publication and mention of the grant of the patent: **23.03.2011**
(21) Application number: 06848667.9
(22) Date of filing: 21.12.2006
(51) Int. Cl.: A61F 2/08, A61B 17/04

(54) **TISSUE GRAFT FIXATION**
FIXIERUNG EINES GEWEBEPFROPFS
FIXATION DE GREFFE DE TISSU

(30) Priority: 22.12.2005 US 313716
(43) Date of publication of application: 03.09.2008
(73) Proprietor: SMITH & NEPHEW, INC., Memphis, Tennessee 38116 (US)
(72) Inventor: LEBEAU, Jason, Fall River, MA 02724 (US); MAHONEY, John, D., Norfolk, MA 02056 (US)
(74) Representative: Guy, Mark Robert
(86) International application number: PCT/US2006/062456
(87) International publication number: WO 2007/073563

(56) References cited:
- WO-A-01/56507
- WO-A1-99/05968
- WO-A2-03/037169
- DE-A1- 4 127 550
- DE-A1- 10 039 767
- FR-A1- 2 726 461
- US-A- 5 057 111
- US-A- 5 306 301
- US-B1- 6 238 418
- DATABASE INSPEC [Online] THE INSTITUTION OF ELECTRICAL ENGINEERS, STEVENAGE, GB; 1995, SANTOS M B ET AL: "Test preparation methodology for high coverage of physical defects in CMOS digital ICs" Database accession no. 5057111

## Description

### Cross-Reference to Related Applications

This application is a PCT International Application of United States Patent Application No. 1 1/313,716 published as US 2007/162125, filed on December 22, 2005.

### Background of the Invention

### 1. Field of the Invention

This invention relates generally to tissue graft fixation, and, more particularly, to a graft fixation member used in fixating a tissue graft.

### 2. Related Art

An anterior cruciate ligament (ACL) that has ruptured and is non-repairable is generally replaced arthroscopically by a tissue graft. The tissue graft can be harvested from a portion of a patellar tendon having so called "bone blocks" at each end, and from the semitendonosis and gracilis. Alternatively, the tissue graft can be formed from synthetic materials or from a combination of synthetic and natural materials. The replacement tissue graft is implanted by securing one end of the tissue graft in a socket formed in a passage within the femur, and passing the other end of the graft through a passage formed in the tibia. Generally, sutures are used to affix each end of the tissue graft to a fastener (e.g., an interference screw or a post), which is then secured to the bone. An example of such a fastener is provided in WO 01/56507.

WO 01/56507 discloses a device for fixing an implant or transplant forming a prosthetic ligament on a bone comprising a base component whose distal portion has a cavity, a locking component configured to be engaged in said cavity, the locking component having a hole in its central portion.

It is also known to use a graft fixation member, e.g., a fixation button, to secure the tissue graft at the femoral cortex, as described in U.S. Patent No. 5,306,301 ("the '301 patent"). When using a fixation button, the femoral passage generally includes a relatively larger diameter portion for receiving the graft, and a smaller diameter, passing channel near the femoral cortex for receiving a length of suture that runs from the tissue graft to the fixation button. By measuring the total length of the femoral passage and the length of the larger diameter portion of the femoral passage, the surgeon determines the appropriate length of suture material for attaching the fixation button to the tissue graft.

There remains a need in the art for a member that allows for the fixation of a tissue graft in a femoral passage having a uniform diameter. This would avoid having to create two passing channels, and thus the use of two separate drills, not to mention measurement of the suture material length. In addition, a fixation member of this type would also allow for drilling of the femoral socket on the outside of the femur. At present, the above discussed larger diameter portion is formed by locating the isometric position at the femoral surface, drilling a guide wire through a tibial passage and into the femur, and then forming a closed-ended femoral socket, having an opening, via use of a drill having an abbreviated drilling head. The smaller diameter portion is then formed by advancing a passing pin, having a canulated drill, to the upper femoral cortex and through the quadriceps and skin. Creation of these portions would not be necessary if a fixation member of the above type was used. Instead, a single portion, having a uniform diameter, could be created via initial drilling from the outside of the femur. This outside drilling would especially be useful in pediatric cases to avoid crossing the epiphysis.

### Summary of the Invention

It is in view of the above problems that the present disclosure was developed. The present disclosure relates to a surgical device used in fixating a tissue graft in a femoral passage.

The present invention relates to a surgical device as claimed hereafter. Preferred embodiments of the invention are set forth in the dependent claims.

In one aspect of the invention, a surgical device includes a member having first and second opposing surfaces. The member includes an intermediate portion in which the first surface has a recessed surface portion. The intermediate portion defines a hole extending from the recessed surface portion to the second surface. End portions extend from the intermediate portion, and each end portion defines a hole extending from the first surface to the second surface. The surgical device includes a second member sized to be received within the recessed surface portion. The intermediate portion defines two additional holes extending from the recessed surface portion to the second surface. The member has a tapered surface portion on the second surface. The intermediate portion hole is elongated.

In another aspect of the present disclosure, a surgical device includes first and second members. The first member has first and second opposing surfaces, a recessed surface portion in the first surface, and a hole extending from the recessed surface portion to the second surface. The second member defines a hole and is configured to be received within the recessed portion of the first member. The holes are positioned to be axially aligned when the second member is received within the recessed portion. The recessed portion is configured to provide a snap-fit or an interference-fit with the second member. The surgical device includes a suture extending through the holes. The first member defines at least two additional holes, for example, a total of five holes. The second member defines three additional holes. The first member has a tapered surface portion on the second surface of the member.

In a further aspect of the present disclosure, the surgical device is used to fixate a tissue graft in a femoral passage having a uniform diameter of between about 6 mm to about 10 mm. In another aspect, the surgical device is used to fixate a tissue graft in a femoral passage having a first passing channel of a first diameter and a second passing channel of a second diameter wherein the second diameter is smaller than the first diameter. In yet a further aspect of the present disclosure, the recessed surface portion includes different shapes and sizes.

A method not, claimed for using the surgical device of the present invention may include locating a tissue fixation device at an opening to a bone hole, the tissue fixation device being coupled to a tissue graft by a flexible member, for example, suture, and increasing the size of the tissue fixation device by placing the tissue fixation device within a larger device having a hole for receiving the flexible member. This aspect may include increasing the size of the tissue fixation device prior to locating the tissue fixation device at the opening.

The surgical device is used to fixate a tissue graft in a femoral passage having a uniform diameter of between about 6 mm to about 10 mm. In another aspect, the surgical device is used to fixate a tissue graft in a femoral passage having a first passing channel of a first diameter and a second passing channel of a second diameter wherein the second diameter is smaller than the first diameter. In yet a further aspect of the present disclosure, the recessed surface portion includes different shapes and sizes.

The present disclosure has several advantages over prior devices and techniques. First, the surgical device allows for the fixation of a tissue graft in a femoral passage having a uniform diameter of between about 6 mm to 10 mm. This avoids the need for using two drills and measuring of the suture material length as is done in the present procedure noted above. Second, the surgical device allows for the continuation of an endoscopic procedure despite perforation of the cortex region. Third, the surgical device allows for drilling on the outside of the femur.

Further features, aspects, and advantages of the present invention, as well as the structure and operation of various embodiments of the present invention, are described in detail below with reference to the accompanying drawings.

### Brief Description of the Drawings

The accompanying drawings, which are incorporated in and form a part of the specification, illustrate the embodiments of the present invention and together with the description, serve to explain the principles of the invention. In the drawings:

Fig. 1 is an illustration of a tissue graft secured within the knee by a graft fixation member.

Fig. 2A is an exploded, perspective view of the graft fixation member of fig. 1.

Fig. 2B is an end view of the graft fixation member of Fig. 1.

Fig. 3A is an end view of a washer of the graft fixation member of Fig. 1.

Fig. 3B is a side view of the washer of Fig. 3A.

Fig. 4A is a perspective view of the graft fixation member of Fig. 1 shown threaded with suture

Fig. 4B is an illustration of the graft fixation member coupled to a tissue graft, with the washer shown in dashed line.

Fig. 5 illustrates drilling a femoral passage within a knee.

Fig. 6 shows engagement of the graft fixation member with a suture retrieval device for pulling the graft fixation member through the femoral passage.

Fig. 7A is perspective view of a washer according to another embodiment.

Fig. 7B is a side view of the washer of Fig. 7A.

Fig. 7C is a sectional view taken along line 7C-7C in Fig. 7B.

Fig. 8 is a side view of a washer according to another embodiment.

Fig. 9 is a side view of a a washer according to another embodiment.

Fig. 10A is perspective view of a washer according to another embodiment.

Fig. 10B is an end view of the washer of Fig. 10A.

Fig. 10C is a sectional view taken along line 10C-10C in Fig. 10B.

### Detailed Description of the Embodiments

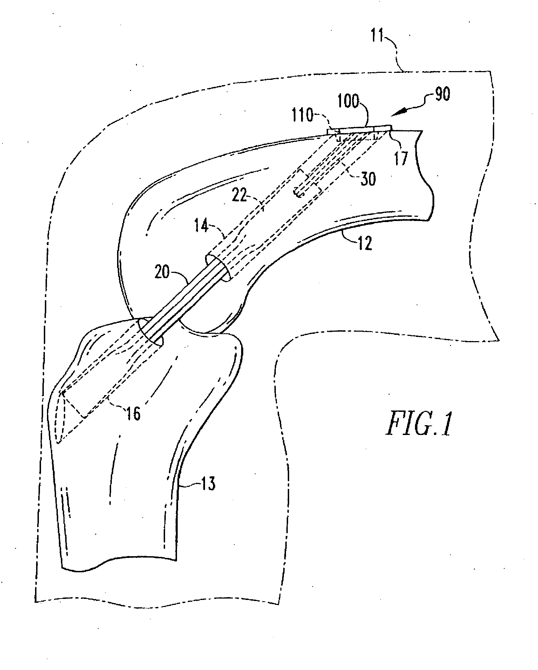

Referring to the accompanying drawings in which like reference numbers indicate like elements, Figure 1 illustrates a fixation member 90 connected to a bone block 22 of a tissue graft 20 by a suture 30, wherein the fixation member 90 is positioned over the opening 17 of a femoral passage 14 to secure the graft 20 in the femoral passage 14. Fixation member 90 includes a second member, e.g., a button 100, received within a first member, e.g., a washer 110. In contrast to techniques that include drilling a passing channel in the femur, such as described in the '301 patent, fixation member 90 enables femoral passage 14 to have a substantially uniform diameter, thus eliminating the added steps of measuring and drilling the relatively smaller passing channel.

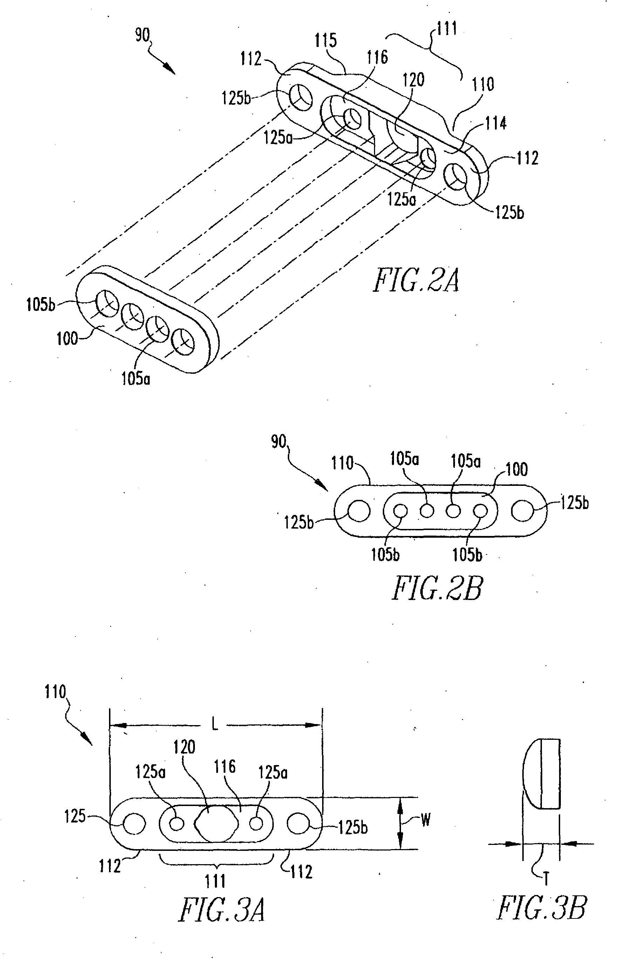

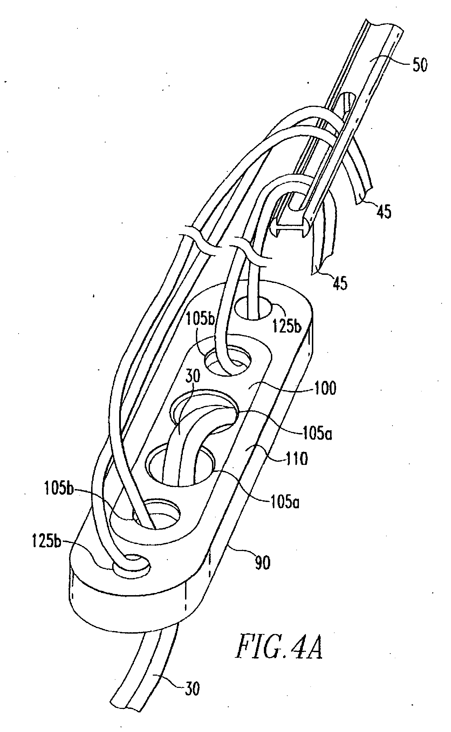

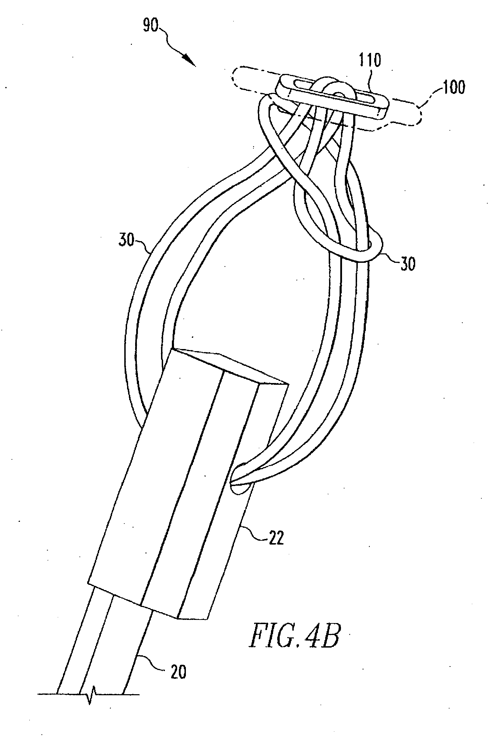

Referring to FIGS. 2A-3B, the washer 110 has a generally elongate shape with an intermediate portion 111 and end portions 112 extending from the intermediate portion 111. Washer 110 has first and second opposing surfaces 114, 115, and the intermediate portion 111 has a recessed surface portion 116 for receiving button 100. Intermediate portion 111 has a hole 120, preferably centrally located, extending from the recessed surface portion 116 to the second surface 115 for receiving the graft tissue suture 30. The washer 110 defines four additional holes, one hole 125b in each of the end portions 112, and two inner holes 125a in intermediate portion 111 on either side of central hole 120, for receiving a pull suture 45 (FIG. 4). Holes 125 extend from the first surface 114 to the second surface 115 of the washer.

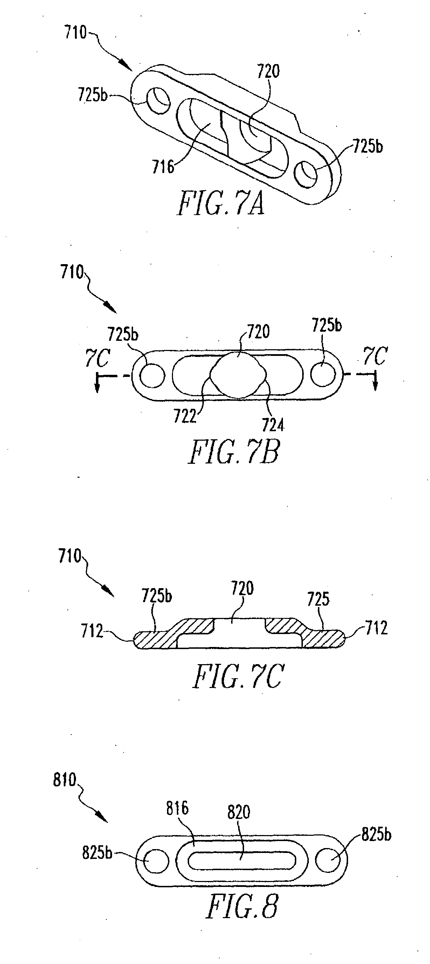

The washer surface 115 is tapered from end portions 112 to intermediate portion 111 (such as shown in FIG. 7C). It is the intermediate portion 111 of the washer that is received within the opening 17 of the femoral passage 14 during use with the washer surface 115 facing the femoral passage. The taper permits fixation member 90 to securely engage the opening 17 of the femoral passage 14 with the washer end portions 112 against the bone surface, while maintaining a low profile relative to bone surface. The taper facilitates a tight fit between the fixation member 90 and passage openings 17 having a range of diameters. Thus, the tapered portion reduces the need to precisely match the size of the graft fixation member with the femoral passage opening. However, the washer need not include the tapered portion.

The button 100 defines four holes. There are two central holes 105a that align with the washer central hole 120, and two end holes 105b that each align with one of the inner washer holes 125a when the button 100 is positioned within the washer recess 116. The elongated washer 110 has a length L, a width W and a thickness T, for example, for an adult femur, the washer 110 has a length of about 15-30 mm, for example, 20 mm, a width of about 6-10 mm, for example, 6mm, and a thickness of about 1-4 mm, for example, 3mm, to bridge the span of a femoral passage 14 and provide sufficient rigidity to adequately tension sutures holding a tissue graft 20 while maintaining a relatively low profile with respect to the surface of the femur 12. The washer recess 116 is dimensioned to receive the button 100 by, for example, a snap-fit or interference-fit, and has a depth corresponding to the thickness of the button such that the button is flush with the surface 114 of the washer 110.

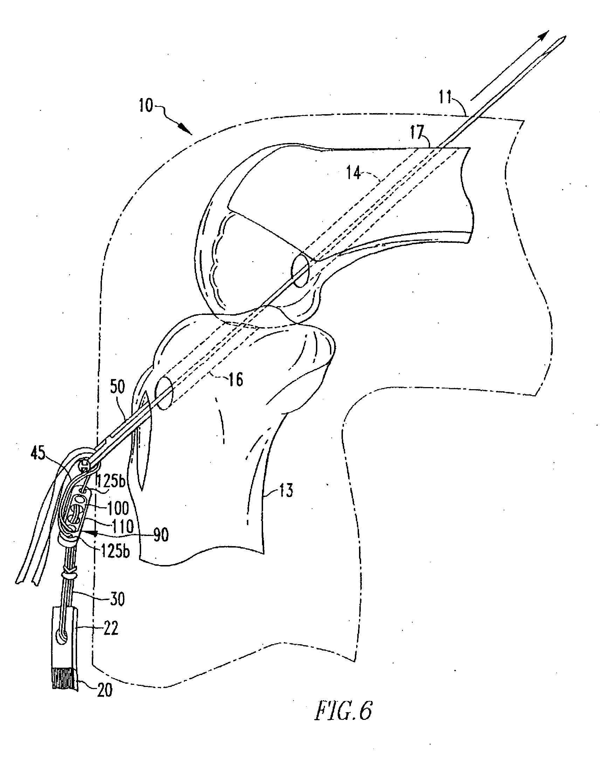

Referring to FIGS. 4A and 4B, the button 100/washer 110 combination is provided with two closed loops of suture 30 extending through washer hole 120 (not shown) and threaded through button holes 105a. Graft fixation member 90 is also provided with one suture 45 threaded through one of the washer holes 125b, back through the adjacent washer hole 125a, and through the corresponding button hole 105b, and a second suture 45 similarly threaded through the holes on the other side of the washer and button. The sutures 45 are then passed through an opening located at the proximal end of a passing pin 50, as more thoroughly described bellow. Alternatively, operating room personnel can thread the sutures 45. Fig. 6 shows an alternative threading of sutures 45, with one suture 45 threaded through each of the washer holes 125b. Here, washer holes 125a and button holes 105b are not used.

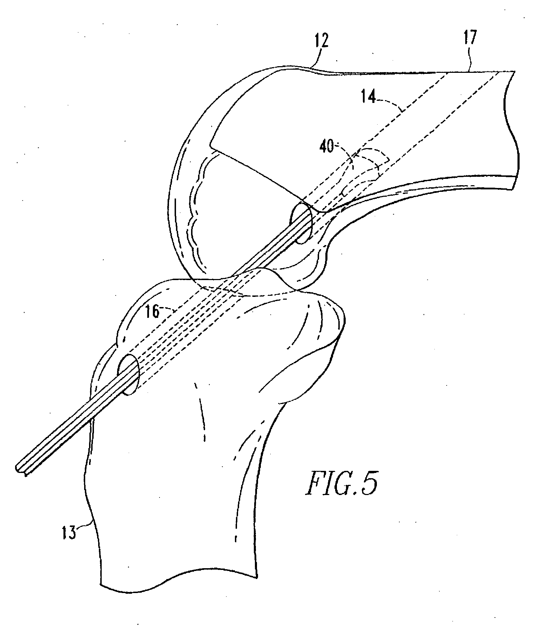

Referring to FIGS. 5 and 6, in use, a femoral hole 17 of substantially uniform diameter is drilled using a drill 40. To pull the graft fixation member 90 and tissue graft 20 through the tibia and femur passages, a suture retrieving device, such as a passing pin 50, is used to removably engage the free ends of sutures 45 and pull the fixation member 90 through the knee 10.

Referring to FIG. 1, once the fixation member 90 has been pulled through the femoral passage 14, the surgeon positions the fixation member 90 transversely to the femoral passage 14 and across the opening 17. To complete the fixation of the tissue graft, the fixation member 90 is secured against the femur 12 by attaching the tissue graft 20 to the tibia 13 and tensioning the tissue graft 20 and the closed-loop suture 30 according to methods described in the '301 patent.

Other embodiments are within the scope of the following claims. For example, other embodiments can include washers appropriately sized and shaped to receive buttons with a variety of geometries. Referring to FIGS. 7A-7C, rather than including both the central hole 120 and suture holes 125a within the recess 116, a washer 710 includes only a central hole 720 within a recess 716 sized and shaped to accommodate suture from the four holes lost and 105b of the button. Hole 720 includes two side extending openings 722, 724 for receiving suture from holes 105b. The washer 710 includes suture holes 725b in end portions 712 of the washer 710.

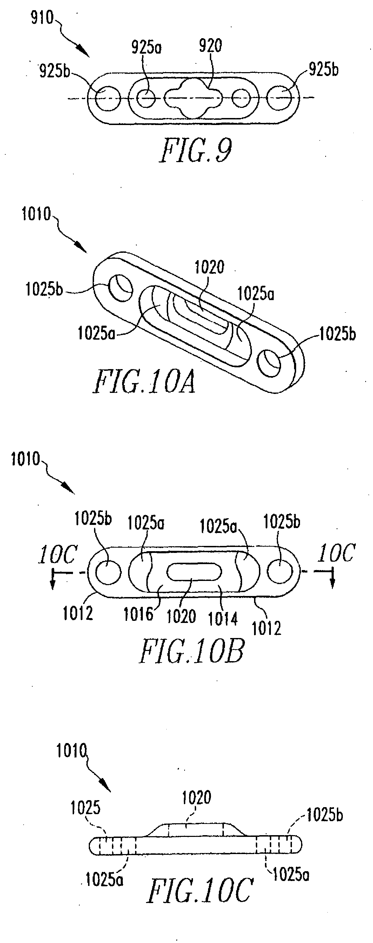

Referring to FIG. 8, in another embodiment, a washer 810 includes only an elongated, central hole 820 within a recess 816, and side holes 825b. Referring to FIG. 9, in another embodiment, a washer 910 includes a central hole 920 and side holes 925b, analogous to holes 720 and 725b in washer 710, but additionally includes holes 925a. In another embodiment (FIGS. 10A-10C), a washer 1010 includes round suture holes 1025b in end portions 1012, and oblong suture holes 1025a at recess portion 1016 adjacent to a central hole 1020.

A kit not claimed, including at least one surgical device having the aforementioned elements is also within the scope of the present disclosure.

Although the aforementioned embodiments have been described in connection with procedures having a single, uniformly-sized femoral passage, the graft fixation member can be applied to procedures in which a first passing channel having a first diameter and a second passing channel having a smaller second diameter are formed in the femoral passage. For example, the creation of a smaller passing channel may be desirable in some patients, such as a child whose bones are growing or an elderly patient without sufficient bone density to accommodate a relatively large femoral passage 14. In these cases, the graft fixation member advantageously increases the surface area of the bone over which tension is applied.

The recess of the washer can be sized and shaped to completely or partially receive the button. Washers can be provided having different sized and/or shaped recesses to accommodate different buttons. For example, the recessed portion 116 of washer 110 can be sized and shaped to receive the fixation buttons described in U.S. Pat. No. 6,533,802 and U.S. Pat. Application No. 10/895,266, filed July 20, 2004, published as US 2005-0038427 A1.

The closed-loop suture 30 can be, but is not limited to, a continuous loop made from polyester, a strand of suture tied in a loop, or a piece of polyester closure tape (e.g. Marselene™ from Ethicon Inc., Cincinnati, Ohio) tied in a loop.

The graft fixation member is formed from a biocompatible material such as titanium or PEEK (Polyetheretherketone).

The fixation member can be used with tissue grafts other than those with bone blocks, for example, harvested tissue graft without bone blocks, and tissue grafts formed from synthetic materials or from a combination of synthetic and natural materials.

In view of the foregoing, it will be seen that the several advantages of the invention are achieved and attained.

The embodiments were chosen and described in order to best explain the principles of the invention and its practical application to thereby enable others skilled in the art to best utilize the invention in various embodiments and with various modifications as are suited to the particular use contemplated.

As various modifications could be made in the constructions and methods herein described and illustrated without departing from the scope of the invention, it is intended that all matter contained in the foregoing description or shown in the accompanying drawings shall be interpreted as illustrative rather than limiting. For example, while Figure 5 illustrates the mount 110 being coupled to the frame 120 through the use of the locking pin 112, other structure and/or methods may be used to temporarily affix these items together. Thus, the breadth and scope of the present invention should not be limited by any of the above-described exemplary embodiments, but should be defined only in accordance with the following claims appended hereto and their equivalents.

## Claims

1. A surgical device (90) for fixating a tissue graft comprising:
a first member (110, 710, 810, 910,1010) having first and second opposing surfaces (114, 115), the first member (110, 710, 810, 910, 1010) including an intermediate portion (111) in which the first surface (114) has a recessed surface portion (116, 716, 816, 1016), the intermediate portion (111) defining a hole (120, 720, 820, 920,1020) extending from the recessed surface portion (116, 716, 816, 1016) to the second surface (115); and
a second member (100) defining a hole (105a), the second member (100) being configured to be received within the recessed portion (116, 716, 816, 1016) of the first member (110, 710, 810, 910, 1010);
**characterised in that** end portions (112, 712, 1012) extend from the intermediate portion (111) of the first member, each end portion (112, 712, 1012) defining a hole (125b, 725b, 825b, 925b,1025b) extending from the first surface (114) to the second surface (115).

2. The surgical device of claim 1, wherein the holes (120, 720, 820, 920, 1020, 105a) of the intermediate portion (111) and second member (100) are positioned to be axially aligned when the second member (100) is received within the recessed portion (116, 716, 816, 1016).

3. The surgical device of any one of claims 1 or 2, further comprising a suture (30) extending through the holes (120, 720, 820, 920, 1020, 105a).

4. The surgical device of any one of claims 1 to 3, wherein the intermediate portion (111) defines two additional holes (125a, 925a, 1025a) extending from the recessed surface portion (116, 1016) to the second surface (115).

5. The surgical device of claim 4, wherein the first member (110, 910, 1010) defines five holes.

6. The surgical device of any one of claims I to 3, wherein the intermediate portion hole (820) comprises an elongated hole.

7. The surgical device of any of the preceding claims, wherein the first member (110, 710, 810, 910, 1010) further comprises a tapered surface portion on the second surface (115) of the member.

8. The surgical device of any of the preceding claims, wherein the recessed portion (116, 716, 816, 1016) is configured to provide a snap-fit with the second member (100),

9. The surgical device of any one of claims 1 to 7, wherein the recessed portion (116, 716, 816, 1016) is configured to provide an interference-fit with the second member (100).

10. The surgical device of any of the preceding claims, wherein the second member (100) defines three additional holes (105a, 105b).

11. The surgical device as in any of the preceding claims wherein the surgical device (90) is used to fixate a tissue graft (20) in a femoral passage (14).

12. The surgical device as in claim 11 wherein the femoral passage (14) is of a uniform diameter.

13. The surgical device as in claim 12 wherein the uniform diameter comprises between about 6 mm to about 10 mm.

14. The surgical device as in any of the preceding claims wherein the recessed surface portion (116, 716, 816, 1016) comprises different shapes and sizes.

15. The surgical device as in any one of claims 1 to 11 wherein the surgical device (90) is used to fixate a tissue graft (20) in a femoral passage (14) having a first passing channel of a first diameter and a second passing channel of a second diameter.

16. The surgical device as in claim 15 wherein the second diameter is smaller than the first diameter.

## Patentansprüche

1. Eine chirurgische Vorrichtung (90) zum Fixieren eines Gewebetransplantats, die Folgendes beinhaltet:
ein erstes Element (110, 710, 810, 910, 1010), das eine erste und eine zweite sich gegenüberliegende Oberfläche (114, 115) aufweist, wobei das erste Element (110, 710, 810, 910, 1010) einen mittleren Abschnitt (111) umfasst, in dem die erste Oberfläche (114) einen ausgesparten Oberflächenabschnitt (116, 716, 816, 1016) aufweist, wobei der mittlere Abschnitt (111) ein Loch (120, 720, 820, 920, 1020) definiert, das sich von dem ausgesparten Oberflächenabschnitt (116, 716, 816, 1016) zu der zweiten Oberfläche (115) erstreckt; und
ein zweites Element (100), das ein Loch (105a) definiert, wobei das zweite Element (100) konfiguriert ist, um innerhalb des ausgesparten Abschnitts (116, 716, 816, 1016) des ersten Elements (110, 710, 810, 910, 1010) aufgenommen zu werden;
**dadurch gekennzeichnet, dass** sich von dem mittleren Abschnitt (111) des ersten Elements Endabschnitte (112, 712, 1012) erstrecken, wobei jeder Endabschnitt (112, 712, 1012) ein Loch (125b, 725b, 825b, 925b, 1025b) definiert, das sich von der ersten Oberfläche (114) zu der zweiten Oberfläche (115) erstreckt.

2. Chirurgische Vorrichtung gemäß Anspruch 1, wobei die Löcher (120, 720, 820, 920, 1020, 105a) des mittleren Abschnitts (111) und des zweiten Abschnitts (100) positioniert sind, um axial ausgerichtet zu sein, wenn das zweite Element (100) innerhalb des ausgesparten Abschnitts (116, 716, 816, 1016) aufgenommen wird.

3. Chirurgische Vorrichtung gemäß einem der Ansprüche 1 oder 2, die ferner ein sich durch die Löcher (120, 720, 820, 920, 1020, 105a) erstreckendes Nahtmaterial (30) beinhaltet.

4. Chirurgische Vorrichtung gemäß einem der Ansprüche 1 bis 3, wobei der mittlere Abschnitt (111) zwei zusätzliche Löcher (125a, 925a, 1025a) definiert, die sich von dem ausgesparten Oberflächenabschnitt (116, 1016) zu der zweiten Oberfläche (115) erstrecken.

5. Chirurgische Vorrichtung gemäß Anspruch 4, wobei das erste Element (110, 910, 1010) fünf Löcher definiert.

6. Chirurgische Vorrichtung gemäß einem der Ansprüche 1 bis 3, wobei das Loch (820) des mittleren Abschnitts ein längliches Loch beinhaltet.

7. Chirurgische Vorrichtung gemäß einem der vorhergehenden Ansprüche, wobei das erste Element (110, 710, 810, 910, 1010) auf der zweiten Oberfläche (115) des Elements ferner einen Abschnitt mit sich verjüngender Oberfläche beinhaltet.

8. Chirurgische Vorrichtung gemäß einem der vorhergehenden Ansprüche, wobei der ausgesparte Abschnitt (116, 716, 816, 1016) konfiguriert ist, um mit dem zweiten Element (100) eine Schnappverbindung bereitzustellen.

9. Chirurgische Vorrichtung gemäß einem der Ansprüche 1 bis 7, wobei der ausgesparte Abschnitt (116, 716, 816, 1016) konfiguriert ist, um mit dem zweiten Element (100) eine Presspassung bereitzustellen.

10. Chirurgische Vorrichtung gemäß einem der vorhergehenden Ansprüche, wobei das zweite Element (100) drei zusätzliche Löcher (1 05a, 105b) definiert.

11. Chirurgische Vorrichtung gemäß einem der vorhergehenden Ansprüche, wobei die chirurgische Vorrichtung (90) verwendet wird, um ein Gewebetransplantat (20) in einem Femurdurchgang (14) zu befestigen.

12. Chirurgische Vorrichtung gemäß Anspruch 11, wobei der Femurdurchgang (14) einen einheitlichen Durchmesser aufweist.

13. Chirurgische Vorrichtung gemäß Anspruch 12, wobei der einheitliche Durchmesser zwischen etwa 6 mm bis etwa 10 mm beinhaltet.

14. Chirurgische Vorrichtung gemäß einem der vorhergehenden Ansprüche, wobei der ausgesparte Oberflächenabschnitt (116, 716, 816, 1016) unterschiedliche Formen und Größen beinhaltet.

15. Chirurgische Vorrichtung gemäß einem der Ansprüche 1 bis 11, wobei die chirurgische Vorrichtung (90) verwendet wird, um ein Gewebetransplantat (20) in einem Femurdurchgang (14) mit einem ersten Durchgangskanal mit einem ersten Durchmesser und einem zweiten Durchgangskanal mit einem zweiten Durchmesser zu befestigen.

16. Chirurgische Vorrichtung gemäß Anspruch 15, wobei der zweite Durchmesser kleiner als der erste Durchmesser ist.

## Revendications

1. Un dispositif chirurgical (90) destiné à fixer un greffon tissulaire comprenant :
un premier élément (110, 710, 810, 910, 1010) présentant des première et deuxième surfaces opposées (114, 115), le premier élément (110, 710, 810, 910, 1010) incluant une portion intermédiaire (111) dans laquelle la première surface (114) présente une portion de surface en renfoncement (116, 716, 816, 1016), la portion intermédiaire (111) définissant un trou (120, 720, 820, 920, 1020) s'étendant de la portion de surface en renfoncement (116, 716, 816, 1016) à la deuxième surface (115) ; et
un deuxième élément (100) définissant un trou (105a), le deuxième élément (100) étant configuré de façon à être reçu au sein de la portion en renfoncement (116, 716, 816, 1016) du premier élément (110, 710, 810, 910, 1010) ;
**caractérisé en ce que** des portions d'extrémité (112, 712, 1012) s'étendent depuis la portion intermédiaire (111) du premier élément, chaque portion d'extrémité (112, 712, 1012) définissant un trou (125b, 725b, 825b, 925b, 1025b) s'étendant de la première surface (114) à la deuxième surface (115).

2. Le dispositif chirurgical de la revendication 1, dans lequel les trous (120, 720, 820, 920, 1020, 105a) de la portion intermédiaire (111) et du deuxième élément (100) sont positionnés de façon à être alignés axialement lorsque le deuxième élément (100) est reçu au sein de la portion en renfoncement (116, 716, 816, 1016).

3. Le dispositif chirurgical de n'importe laquelle des revendications 1 ou 2, comprenant de plus un fil de suture (30) s'étendant à travers les trous (120, 720, 820, 920, 1020, 105a).

4. Le dispositif chirurgical de n'importe laquelle des revendications 1 à 3, dans lequel la portion intermédiaire (111) définit deux trous supplémentaires (125a, 925a, 1025a) s'étendant de la portion de surface en renfoncement (116, 1016) à la deuxième surface (115).

5. Le dispositif chirurgical de la revendication 4, dans lequel le premier élément (110, 910, 1010) définit cinq trous.

6. Le dispositif chirurgical de n'importe laquelle des revendications 1 à 3, dans lequel le trou (820) de la portion intermédiaire comprend un trou allongé.

7. Le dispositif chirurgical de n'importe lesquelles des revendications précédentes, dans lequel le premier élément (110, 710, 810, 910, 1010) comprend de plus une portion de surface effilée sur la deuxième surface (115) de l'élément.

8. Le dispositif chirurgical de n'importe lesquelles des revendications précédentes, dans lequel la portion en renfoncement (116, 716, 816, 1016) est configurée de façon à fournir un emboîtement-pression avec le deuxième élément (100).

9. Le dispositif chirurgical de n'importe laquelle des revendications 1 à 7, dans lequel la portion en renfoncement (116, 716, 816, 1016) est configurée de façon à fournir un emboîtement par ajustement serré avec le deuxième élément (100).

10. Le dispositif chirurgical de n'importe lesquelles des revendications précédentes, dans lequel le deuxième élément (100) définit trois trous supplémentaires (105a, 105b).

11. Le dispositif chirurgical tel que dans n'importe lesquelles des revendications précédentes dans lequel le dispositif chirurgical (90) est utilisé pour fixer un greffon tissulaire (20) dans un passage fémoral (14).

12. Le dispositif chirurgical tel que dans la revendication 11 dans lequel le passage fémoral (14) est d'un diamètre uniforme.

13. Le dispositif chirurgical tel que dans la revendication 12 dans lequel le diamètre uniforme est compris entre environ 6 mm et environ 10 mm.

14. Le dispositif chirurgical tel que dans n'importe lesquelles des revendications précédentes dans lequel la portion de surface en renfoncement (116, 716, 816, 1016) comprend différentes configurations et tailles.

15. Le dispositif chirurgical tel que dans n'importe laquelle des revendications 1 à 11 dans lequel le dispositif chirurgical (90) est utilisé pour fixer un greffon tissulaire (20) dans un passage fémoral (14) présentant un premier canal de passage d'un premier diamètre et un deuxième canal de passage d'un deuxième diamètre.

16. Le dispositif chirurgical tel que dans la revendication 15 dans lequel le deuxième diamètre est plus petit que le premier diamètre.
